(19) Europäisches Patentamt
European Patent Office
Office européen des brevets

(11) Veröffentlichungsnummer: **0 029 966 A2**

(12) # EUROPÄISCHE PATENTANMELDUNG

(21) Anmeldenummer: 80107221.6

(22) Anmeldetag: 20.11.80

(51) Int. Cl.³: C 07 D 501/36
A 61 K 31/545

(30) Priorität: 29.11.79 CH 10601/79

(43) Veröffentlichungstag der Anmeldung: 10.06.81 Patentblatt 81/23

(84) Benannte Vertragsstaaten: CH DE FR GB IT LI

(71) Anmelder: F. HOFFMANN-LA ROCHE & CO. Aktiengesellschaft

CH-4002 Basel(CH)

(72) Erfinder: Montavon, Marc, Dr.
Realpstrasse 72
CH-4054 Basel(CH)

(72) Erfinder: Reiner, Roland, Dr.
Rheinfelderstrasse 8
CH-4058 Basel(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Cephalosporinderivate, entsprechende pharmazeutische Präparate, die Herstellung der Cephalosporinderivate, entsprechende Ausgangsverbindungen sowie die Verwendung der Cephalosporinderivate bei der Behandlung von Krankheiten.**

(57) Cephalosporinderivate der allgemeinen Formel

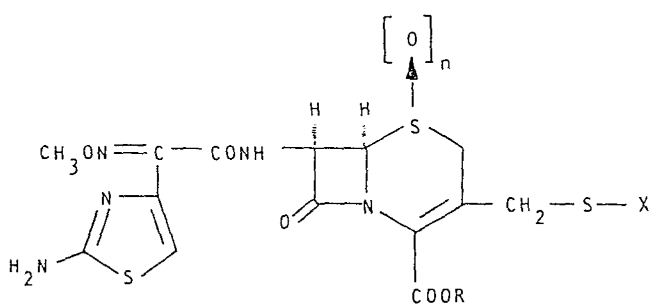

1

in der X eine der Gruppen

./...

EP 0 029 966 A2

Croydon Printing Company Ltd.

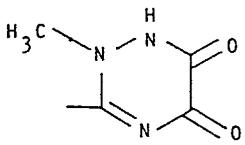

(a),

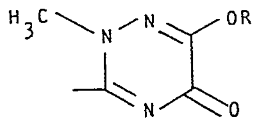

(b)

bedeutet, R Wasserstoff oder eine leicht hydrolysierbare Ester- bzw. Aethergruppe und n die Zahl 1 oder 2 darstellt, sowie Salze dieser Verbindungen und Hydrate dieser Verbindungen bzw. Salze.

Ferner entsprechende pharmazeutische Präparate, die Herstellung der Cephalosporinderivate, entsprechende Ausgangsverbindungen sowie die Verwendung der Cephalosporinderivate bei der Behandlung von Krankheiten insbesondere von Infektionskrankheiten.

F.Hoffmann-La Roche & Co.Aktiengesellschaft, Basel/Schweiz

RAN 4410/139

Cephalosporinderivate, entsprechende pharmazeutische Präparate, die Herstellung der Cephalosporinderivate, entsprechende Ausgangsverbindungen sowie die Verwendung der Cephalosporinderivate bei der Behandlung von Krankheiten

Die vorliegende Erfindung betrifft neue Acylderivate, und zwar Cephalosporinderivate der allgemeinen Formel

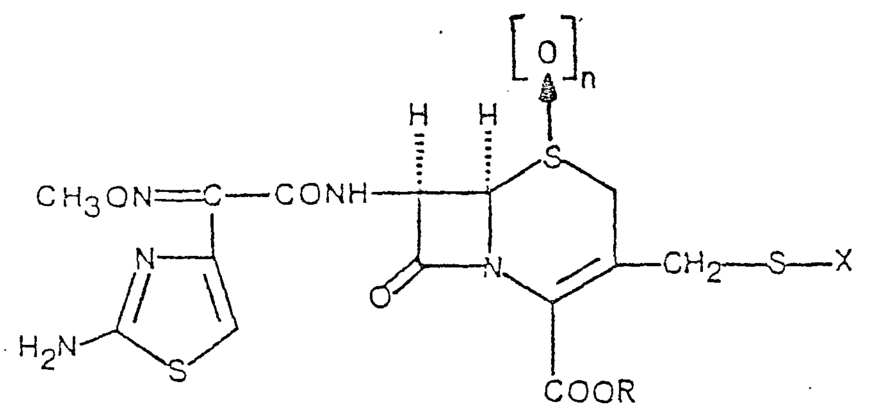

I

in der X eine der Gruppen

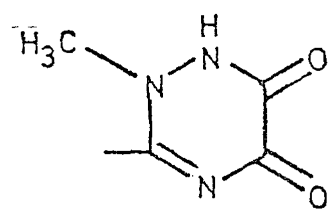

(a)

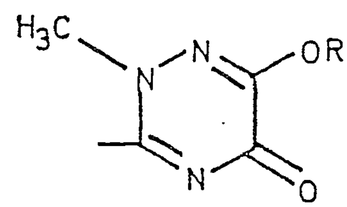

(b)

bedeutet, R Wasserstoff oder eine leicht hydrolysierbare Ester- bzw. Aethergruppe und n die Zahl 1 oder 2 darstellt,

sowie Salze dieser Verbindungen und Hydrate dieser Verbindungen bzw. Salze.

Mn/ 16.10.80

Falls R in der Gruppe (b) Wasserstoff darstellt, liegt diese Gruppe in tautomerem Gleichgewicht mit der Gruppe (a) vor.

Steht n in Formel I für die Zahl 1, liegen die Verbindungen als Sulfoxide vor. Das Sauerstoffatom der Sulfoxidgruppe kann entweder α-ständig (R-Konfiguration) oder ß-ständig (S-Konfiguration) sein oder aus Mischungen dieser beiden Konfigurationen bestehen. Steht n in Formel I für die Zahl 2, liegen die Verbindungen als Sulfone (Dioxide) vor.

Als leicht hydrolysierbare Estergruppen R in den Verbindungen der Formel I sind R-Gruppen an der Carboxylfunktion zu verstehen, welche in Form einer leicht hydrolysierbaren Estergruppe vorliegen. Beispiele solcher Estergruppen, die herkömmlicher Art sein können, sind niederes Alkanoyloxyalkyl, z.B. Acetoxymethyl, Pivaloyloxymethyl, 1-Acetoxyäthyl und 1-Pivaloyloxyäthyl; niederes Alkoxycarbonyloxyalkyl, z.B. Methoxycarbonyloxymethyl, 1-Aethoxycarbonyloxyäthyl und 1-Isopropoxycarbonyloxyäthyl; Lactonylreste, z.B. Phthalidyl und Thiophthalidyl; niederes Alkoxymethyl, z.B. Methoxymethyl; und niederes Alkanoylaminomethyl, z.B. Acetamidomethyl.

Als leicht hydrolysierbare Aethergruppen R in den Verbindungen der Formel I sind R-Gruppen an der enolischen Funktion der 2,5-Dihydro-6-hydroxy-2-methyl-5-oxo-as-triazin-3-yl-gruppe (b) zu verstehen, welche in Form einer leicht hydrolysierbaren Aethergruppe vorliegen. Als Aethergruppen kommen die gleichen Gruppen in Betracht, wie sie oben bereits für die leicht hydrolysierbaren Estergruppen erwähnt wurden. Vertreter solcher Aether sind also beispielsweise die niederen Alkanoyloxyalkyläther, z.B. der Acetoxymethyl-, Pivaloyloxymethyl-, 1-Acetoxyäthyl- und 1-Pivaloyloxyäthyläther; die niederen Alkoxycarbonyloxyalkyläther, z.B. der Methoxycarbonyloxymethyl-,

1-Aethoxycarbonyloxyäthyl- und 1-Isopropoxycarbonyloxyäthyläther; die Lactonyläther, z.B. der Phthalidyl- und Thiophthalidyläther; die niederen Alkoxymethyläther, z.B. der Methoxymethyläther; und die niederen Alkanoylaminomethyläther, z.B. der Acetamidomethyläther.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Monosalze oder auch Disalze sein. Die zweite Salzbildung kann in Verbindungen mit dem Hydroxyrest der 2,5-Dihydro-6-hydroxy-2-methyl-5-oxo-as-triazin-3-yl-gruppe auftreten.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Mono-arylsulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die Verbindungen der Formel I (einschliesslich deren Salze, leicht hydrolysierbare Ester und Aether) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-iso-

meren Form (Z-Form)

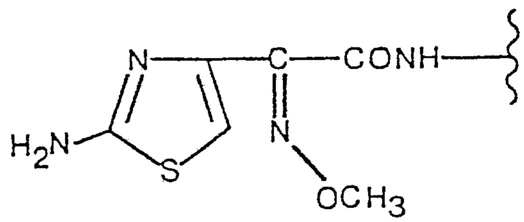

oder in der anti-isomeren Form (E-Form)

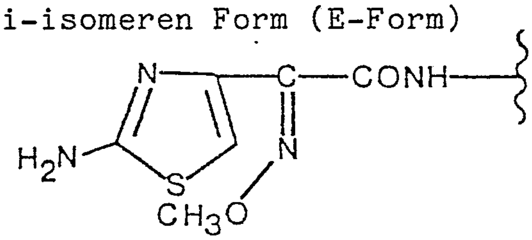

bzw. als Gemische dieser beiden Formen vorliegen. Bevorzugt ist die syn-isomere Form bzw. Gemische, in denen die syn-isomere Form überwiegt.

Bevorzugte Produkte sind

(6R,7R)-7-/2-(2-Amino-4-thiazolyl)-2-[(Z-methoxyimino]-acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure-5-(S)-oxid und dessen Salze sowie die entsprechenden Hydrate;

Methylen (6R,7R)-7-/2-(2-Amino-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid und dessen Salze sowie die entsprechenden Hydrate;

(6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5,5-dioxid und dessen Salze sowie die entsprechenden Hydrate;

Die obigen Acylderivate werden erfindungsgemäss dadurch hergestellt, dass man

a) eine Verbindung der allgemeinen Formel

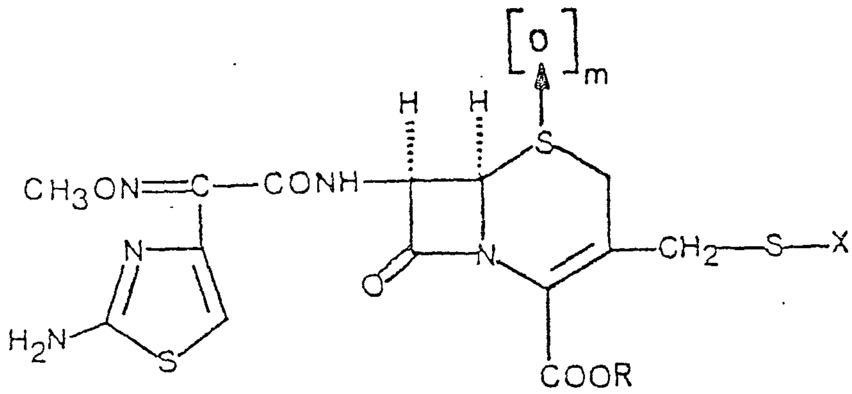

II

in der X und R die oben gegebene Bedeutung haben und m die Zahl 0 oder 1 darstellt,
oder ein Salz dieser Verbindung oxidiert, oder dass man

b) in einer Verbindung der allgemeinen Formel

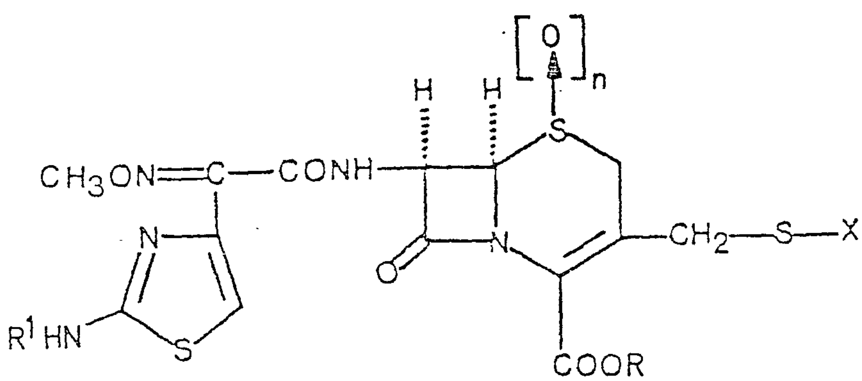

III

in der X, R und n die oben gegebene Bedeutung haben und $R^1$ eine abspaltbare Schutzgruppe darstellt,
oder in einem Salz dieser Verbindung die Schutzgruppe $R^1$ abspaltet, oder dass man

c) ein Halogenid der allgemeinen Formel

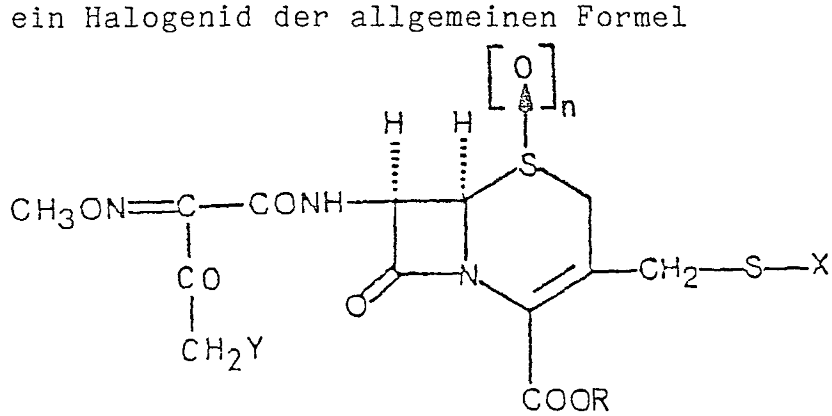

IV

in der X, R und n die oben gegebene Bedeutung haben und Y ein Halogenatom darstellt,
oder ein Salz dieser Verbindung mit Thioharnstoff umsetzt, oder dass man

d) eine Verbindung der allgemeinen Formel

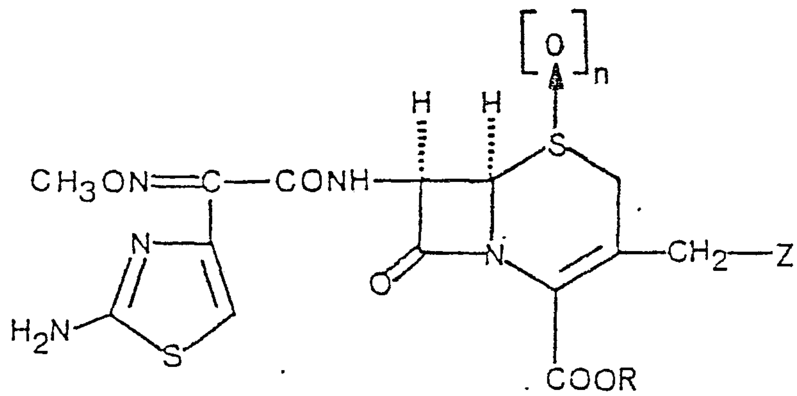

V

in der R und n die oben gegebene Bedeutung haben und Z eine austretende Gruppe darstellt,
oder ein Salz dieser Verbindung mit einem Thiol der allgemeinen Formel

HS-X VI

in der X die oben gegebene Bedeutung hat,
umsetzt, oder dass man

e) zur Herstellung von leicht hydrolysierbaren Estern bzw. Aethern der Formel I, d.h. worin R eine leicht hydrolysierbare Ester- bzw. Aethergruppe darstellt, eine Carbonsäure bzw. ein Enol der Formel I einer entsprechenden Veresterung bzw. Verätherung unterwirft, oder dass man

f) zur Herstellung von Salzen und Hydraten einer Verbindung der Formel I bzw. von Hydraten dieser Salze eine Verbindung der Formel I in ein Salz oder Hydrat bzw. ein Hydrat dieses Salzes überführt.

Die Ausgangsverbindungen der Formel II, worin m die

Zahl 0 darstellt, d.h. ohne Sauerstoff am Ring-Schwefel, können beispielsweise erhalten werden, indem man zunächst eine Zwischenverbindung der allgemeinen Formel

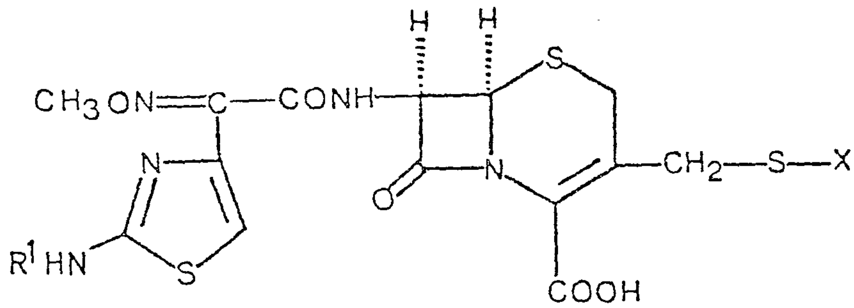

VII

in der X die oben gegebene Bedeutung hat, $R^1$ eine abspaltbare Schutzgruppe darstellt und die Carboxygruppe in geschützter Form vorliegen kann,

herstellt und zwar durch Umsetzung einer Verbindung der allgemeinen Formel

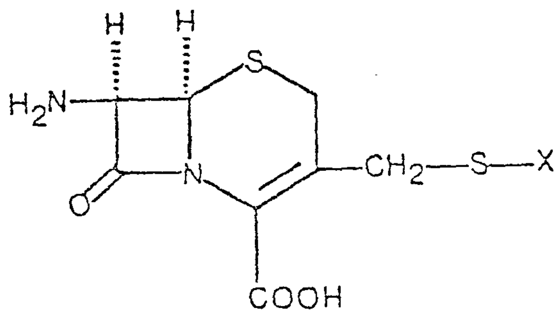

VIII

in der X die oben gegebene Bedeutung hat und die Carboxygruppe und/oder die Aminogruppe in geschützter Form vorliegen kann,

mit einer Säure der allgemeinen Formel

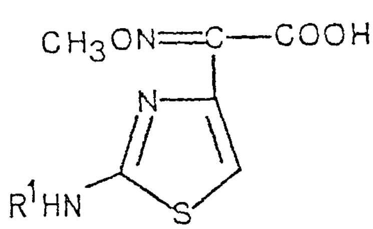

IX

in der $R^1$ die oben gegebene Bedeutung hat,

oder mit einem reaktionsfähigen funktionellen Derivat dieser Säure, wonach man eine allenfalls vorhandene Carboxyschutzgruppe gegebenenfalls abspaltet.

Die in der Zwischenverbindung der Formel VII vorhandene Carboxygruppe kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden. Mögliche $R^1$-Schutzgruppen in den Verbindungen der Formeln VII und IX sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Auch die in der 7-Aminoverbindung der Formel VIII vorhandene Carboxygruppe kann wahlweise geschützt sein, und zwar in der oben für die herzustellende Zwischenverbindung der Formel VII erläuterten Weise. Die Aminogruppe der Verbindung der Formel VIII kann z.B. durch eine Silylschutzgruppe, wie Trimethylsilyl, geschützt sein.

Als reaktionsfähige funktionelle Derivate von Säuren der Formel IX kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel VIII mit der Säure der Formel IX oder einem reaktionsfähigen funktionellen Derivat davon kann in an sich bekannter Weise durchgeführt werden. So kann man z.B. eine freie Säure der Formel IX mit einem der erwähnten Ester entsprechend Formel VIII mittels eines Carbodiimids, wie

N,N-Dicyclohexylcarbodiimid, in einem inerten Lösungsmittel, wie Essigester, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer weiteren Ausführungsform wird ein Säurehalogenid, vorzugsweise das Chlorid einer Säure der Formel IX mit dem Amin der Formel VIII umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggf. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel VIII wird in wasserfreiem Medium gearbeitet.

Die Umsetzung der 7-Aminoverbindung der Formel VIII mit der Säure der Formel IX oder einem reaktionsfähigen funktionellen Derivat davon kann zweckmässig bei Temperaturen zwischen etwa -40°C und Zimmertemperatur, beispielsweise bei etwa 0-10°C, erfolgen.

Die Zwischenverbindungen der Formel VII können auch durch Thiolierung hergestellt werden, und zwar indem man eine Verbindung der allgemeinen Formel

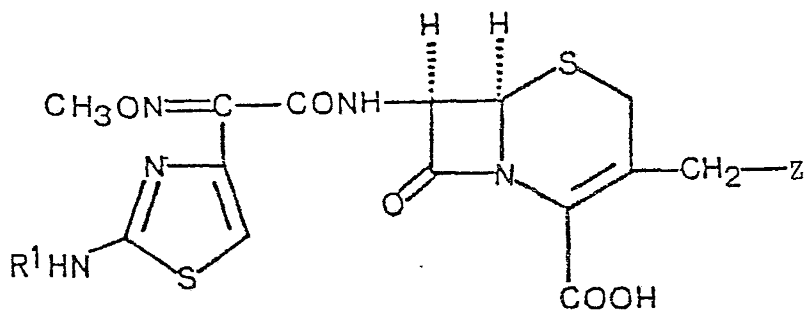

X

in der $R^1$ die oben gegebene Bedeutung hat, Y eine austretende Gruppe darstellt und die Carboxygruppe in geschützter Form vorliegen kann,
oder ein Salz davon mit einem Thiol der allgemeinen Formel

HS-X VI

in der X die oben gegebene Bedeutung hat,
umsetzt und eine allenfalls vorhandene Carboxyschutzgruppe gegebenenfalls abspaltet.

Als austretende Gruppe Y einer Verbindung der Formel X kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage. Die Verbindung X kann an der Carboxygruppe in der für die Zwischenverbindung der Formel VII erläuterten Weise geschützt sein.

Die Umsetzung der Verbindung der Formel X mit dem Thiol der Formel VI kann in an sich bekannter Weise, z.B. bei einer Temperatur zwischen etwa 40 und 80°C, zweckmässig bei etwa 60°C, in einem polaren Lösungsmittel, beispielsweise in einem Alkohol, wie z.B. in einem niederen Alkanol, wie Aethanol, n-Propanol und dgl., in Dimethylformamid oder Dimethylsulfoxid, vorzugsweise in Wasser oder in einer Pufferlösung mit einem pH von etwa 6 bis 7, vorzugsweise 6,5, durchgeführt werden.

Eine erhaltene Säure der Formel VII kann in üblicher Weise in ein Salz übergeführt werden, z.B. analog der nachstehend beschriebenen Ueberführung der Carbonsäuren I in ihre Salze.

In der in obiger Weise erhaltenen Zwischenverbindung der Formel VII bzw. in einem Salz davon wird nun zwecks Erhalts der gewünschten Ausgangsverbindungen der Formel II, worin m die Zahl 0 darstellt, die Aminoschutzgruppe $R^1$ abgespalten. Durch saure Hydrolyse abspaltbare Schutzgruppen werden vorzugsweise mit Hilfe einer niederen Alkancarbonsäure, die ggf. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa $0^oC$ bis $+40^oC$. Alkalisch abspaltbare Schutzgruppen werden im allgemeinen mit verdünnter wässriger Lauge bei $0^oC$ bis $30^oC$ hydrolysiert. Die Chloracetyl-, Bromacetyl- und Jodacetyl-Schutzgruppen können mittels Thioharnstoff in saurem, neutralem oder alkalischem Milieu bei etwa $0-30^oC$ abgespalten werden. Hydrogenolytische Abspaltung (z.B. Abspaltung von Benzyl) ist hier ungeeignet, da bei der Hydrogenolyse die Oximfunktion zur Aminogruppe reduziert wird.

Nach Abspaltung der Aminoschutzgruppe $R^1$ kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lactonyl-, Alkoxymethyl- und Alkanoylaminomethylester werden vorzugsweise enzymatisch mit Hilfe einer geeigneten Esterase (bei etwa $20-40^oC$) abgespalten. Wenn die Carboxylgruppe durch Salzbildung (z.B. mit Triäthylamin) geschützt

ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Eine erhaltene Säure der Formel II kann in üblicher Weise in ein Salz übergeführt werden, z.B. analog der nachstehend beschriebenen Ueberführung der Carbonsäuren I in ihre Salze.

Die Variante a) des erfindungsgemässen Verfahrens, die Oxidation der Ausgangsverbindung der Formel II, worin m die Zahl 0 darstellt, bzw. eines deren Salze, erfolgt durch Behandlung mit einem organischen oder anorganischen Oxidationsmittel. Als Oxidationsmittel dienen verschiedene, Sauerstoff leicht abgebende Verbindungen, wie z.B. organische Peroxide, z.B. monosubstituierte organische Peroxide, wie $C_1$-$C_4$ Alkyl- oder Alkanoylhydroperoxide, wie t-Butylhydroperoxid, Perameisensäure, Peressigsäure, sowie phenylsubstituierte Derivate dieser Hydroperoxide, wie Cumolhydroperoxid, Perbenzoesäure. Der Phenylsubstituent kann gegebenenfalls eine weitere niedere Gruppe, z.B. $C_1$-$C_4$ Alkyl oder Alkoxy, Halogen oder Carboxygruppe tragen, z.B. 4-Methylperbenzoesäure, 4-Methoxyperbenzoesäure, 3-Chlorperbenzoesäure, Monoperphthalsäure. Als Oxidationsmittel können auch verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol, Aethanol, oder einer niederen, ggf. halogenierten Alkancarbonsäure, z.B. Ameisensäure, Essigsäure, Trifluoressigsäure. Die Reaktionstemperatur bewegt sich vornehmlich im Bereiche von -20°C bis +50°C.

Bei Verwendung von äquimolaren Mengen Oxidationsmittel bzw. leichtem Ueberschuss im Verhältnis zum Ausgangsmaterial erhält man vornehmlich das entsprechende Sulfoxid der Formel I, d.h. worin n die Zahl 1 darstellt. Erhöht man die Menge des Oxidationsmittels auf das doppelte stöchiometrische Verhältnis oder mehr, bildet sich das entsprechende Sulfon der Formel I, d.h. worin n die Zahl 2 darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Die Herstellung der Ausgangsverbindung der Formel III kann durch Oxidation der oben erwähnten Zwischenverbindung der Formel VII erfolgen. Die Reaktionsbedingungen sind im wesentlichen die gleichen wie für die oben beschriebene Oxidation der Ausgangsverbindungen der Formel II.

Eine erhaltene Säure der Formel III kann in üblicher Weise in ein Salz übergeführt werden, z.B. analog der nachstehend beschriebenen Ueberführung der Carbonsäuren I in ihre Salze.

Die Variante b) des erfindungsgemässen Verfahrens, die Abspaltung der Aminoschutzgruppe $R^1$ in einer Ausgangsverbindung der Formel III bzw. in einem ihrer Salze, erfolgt im wesentlichen wie oben beschrieben für die Abspaltung der Aminoschutzgruppe der Zwischenverbindung der Formel VII.

Die Herstellung der Ausgangsverbindung der Formel IV erfolgt beispielsweise, indem man das oben beschriebene primäre Amin der Formel VIII mit einer halogenierten Carbonsäure der allgemeinen Formel

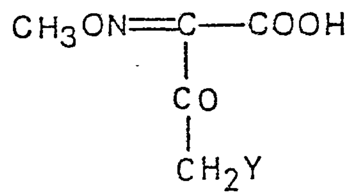

XI

in der Y ein Halogenatom darstellt, oder mit einem reaktionsfähigen Derivat dieser Verbindung umsetzt. Die halogenierte Carbonsäure der Formel XI wird entweder in freier Form eingesetzt in Gegenwart eines Kondensationsmittels, z.B. eines N,N'-disubstituierten Carbodiimides, wie N,N'-Dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z.B. mit Monomethyl- oder Monoisopropylcarbonat, oder in Form eines aktivierten Esters, wie des p-Nitrophenylesters, 2,4-Dinitrophenylesters, N-Hydroxysuccinimidester oder N-Hydroxyphthalimidesters. Die Reaktion erfolgt im allgemeinen in einem inerten organischen Lösungsmittel, z.B. in einem halogenierten Kohlenwasserstoff, wie Chloroform, Dichlormethan, Tetrachlorkohlenstoff, in einem Aether, z.B. Tetrahydrofuran, Dioxan, in Dimethylformamid, Dimethylacetamid, Wasser oder Mischungen davon. Die Reaktionstemperatur liegt vornehmlich im Bereich von etwa -50 bis +40°C, vorzugsweise bei etwa -10 bis +10°C.

Durch die oben beschriebene Umsetzung der Verbindungen der Formel VIII mit den halogenierten Carbonsäuren der Formel XI bzw. mit reaktionsfähigen Derivaten davon erhält man Zwischenverbindungen der allgemeinen Formel

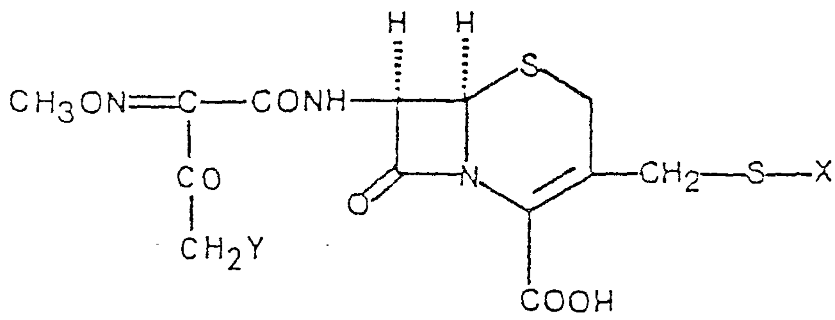

XII

in der X und Y die oben gegebene Bedeutung haben und die Carboxygruppe in geschützter Form vorliegen kann.

Diese Zwischenverbindungen werden durch Oxidation in die gewünschten Ausgangsverbindungen der Formel IV übergeführt, wobei die Reaktionsbedingungen im wesentlichen die gleichen sind wie bei der oben beschriebenen Oxidation der Ausgangsverbindungen der Formel II. Eine allfällige Schutzgruppe an der Carboxyfunktion kann erwünschtenfalls abgespalten werden analog der Abspaltung der Carboxyschutzgruppen, wie sie oben bei der Ueberführung der Zwischenverbindungen VII in die Ausgangsverbindungen der Formel II beschrieben wurde.

Eine erhaltene Säure der Formel IV kann in üblicher Weise in ein Salz übergeführt werden, z.B. analog der nachstehend beschriebenen Ueberführung der Carbonsäuren I in ihre Salze.

Die erfindungsgemässe Umsetzung des Halogenids der Formel IV bzw. eines Salzes davon mit Thioharnstoff gemäss Variante c) des erfindungsgemässen Verfahrens verläuft vorzugsweise in einem Lösungsmittel, wie z.B. in einem niederen Alkanol, z.B. Aethanol, in einem niederen Keton, wie Aceton, in einem Aether, wie Tetrahydrofuran, Dioxan, in Dimethylformamid, Dimethylacetamid, in Wasser oder in Mischungen davon. Die Rektionstemperatur liegt im allgemeinen im Bereich von etwa 0°C

bis 60°C, vorzugsweise bei Zimmertemperatur. Als Halogenid der Formel IV kann das Chlorid, Bromid, Fluorid oder Jodid eingesetzt werden, bevorzugt verwendet man das Chlorid oder das Bromid. Es kann die freie Säure der Formel IV eingesetzt werden, wahlweise aber auch ein Salz davon, wobei die gleichen Salze wie die oben erläuterten Salze der Verbindungen der Formel I in Betracht kommen.

Die Ausgangsverbindung der Formel V kann durch Oxidation einer Verbindung der Formel X, im wesentlichen wie oben beschrieben für die Oxidation der Ausgangsverbindungen der Formel II, und Abspaltung der Aminoschutzgruppe $R^1$, im wesentlichen wie oben beschrieben für die Abspaltung dieser Gruppe von Verbindungen der Formel VII, hergestellt werden.

Eine erhaltene Säure der Formel V kann in üblicher Weise in ein Salz übergeführt werden, z.B. analog der nachstehend beschriebenen Ueberführung der Carbonsäuren I in ihre Salze.

Die Variante d) des erfindungsgemässen Verfahrens, d.h. die Thiolierung der Ausgangsverbindungen der Formel V bzw. eines deren Salze mit einem Thiol der Formel VI, kann im wesentlichen in der gleichen Weise wie die oben beschriebene Thiolierung der Verbindungen der Formel X erfolgen.

Zur Herstellung der leicht hydrolysierbaren Ester der Carbonsäuren der Formel I gemäss Variante e) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin beschleunigt werden. Bei Vorhandensein der 2,5-Dihydro-6-hydroxy-2-methyl-5-oxo-as-triazin-3-yl-

gruppe X mit ihrer enolischen Funktion wird diese unter Bildung eines entsprechenden, leicht hydrolysierbaren Aethers veräthert. Vorzugsweise verwendet man dabei einen Ueberschuss an dem entsprechenden Halogenid. Die Veresterungs-/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Die Herstellung der Salze und Hydrate der Verbindungen der Formel I bzw. der Hydrate dieser Salze gemäss Variante f) des erfindungsgemässen Verfahrens kann in an sich bekannter Weise erfolgen, z.B. durch Umsetzung der Carbonsäure der Formel I mit einer äquivalenten Menge der gewünschten Base, zweckmässig in einem Lösungsmittel, wie Wasser oder in einem organischen Lösungsmittel, wie Aethanol, Methanol, Aceton und anderen mehr. Bei Verwendung eines zweiten Aequivalents an Base erfolgt Salzbildung auch an einer allenfalls vorhandenen tautomeren Enolform (2,5-Dihydro-6-hydroxy-2-methyl-5-oxo-as-triazin-3-ylgruppe X), wobei ein Disalz entsteht. Die Temperatur der Salzbildung ist nicht kritisch, sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Aether oder Salze davon) einer feuchten Atmosphäre, z.B. bei etwa +10°C bis +40°C, ausgesetzt werden.

Die oben verwendeten 7-Aminoverbindungen der Formel VIII können ausgehend von einer Verbindung der Formel

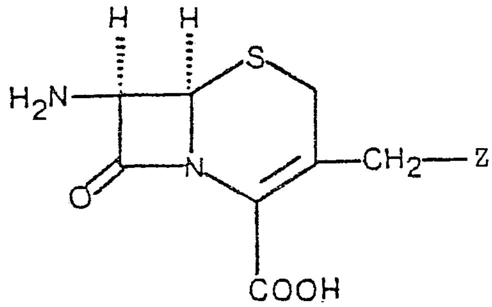

XIII

in der Y eine austretende Gruppe darstellt und die Carboxygruppe in geschützter Form vorliegen kann, mit einem Thiol der Formel VI hergestellt werden. Die Umsetzung kann unter den gleichen Bedingungen wie denjenigen erfolgen, wie sie für die Umsetzung der Ausgangsverbindungen X mit VI beschrieben wurden. Andererseits können die Zwischenverbindungen der Formel X ausgehend von einer Verbindung der Formel XIII und einer Säure der Formel IX oder einem reaktionsfähigen funktionellen Derivat davon unter den gleichen Bedingungen hergestellt werden, wie sie für die Umsetzung der Verbindungen der Formeln VIII und IX beschrieben wurden.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I sowie die entsprechenden Salze bzw. die Hydrate dieser Produkte sind antibiotisch, insbesondere bakterizid wirksam. Sie besitzen ein breites Wirkungsspektrum gegen Gram-positive und Gram-negative Mikroorganismen, einschliesslich verschiedene ß-Lactamase-bildende Gram-negative Bakterien, wie z.B. Escherichia coli, Serratia marcescens, Proteus- und Enterobacter-Spezies. Die Verbindungen zeichnen sich durch besonders hohe β-Lactamase-Stabilität aus.

Die Verbindungen der Formel I sowie die entsprechenden Salze bzw. die Hydrate dieser Produkte können zur

Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erwähnten Produkte wurden folgende repräsentative Vertreter getestet:

Produkt A: Das Dinatriumsalz des (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure-5-(S)-oxids

Produkt B: Das Dinatriumsalz des (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure-5,5-dioxids

Aktivität in vitro: Mindesthemmkonzentration (µg/ml)

| Erreger | | A | B |
|---|---|---|---|
| GRAMPOSITIVE | | | |
| Streptococcus pyogenes | | 1,6 | 3,1 |
| GRAMNEGATIVE | | | |
| Escherichia coli | Stamm 1 | 0,025 | 0,05 |
| | Stamm 2 | 0,19 | 0,39 |
| | Stamm 3 | 0,10 | 0,19 |
| | Stamm 4 | 0,10 | 0,39 |
| Enterobacter cloacae | Stamm 1 | 3,1 | 6,3 |
| | Stamm 2 | 0,19 | 0,39 |
| Enterobacter aerogenes | Stamm 1 | 0,19 | 0,39 |
| | Stamm 2 | 0,10 | 0,10 |
| Klebsiella pneumoniae | Stamm 1 | 0,10 | 0,39 |
| | Stamm 2 | 0,025 | 0,19 |
| | Stamm 3 | 0,10 | 0,19 |
| Citrobacter freundii | Stamm 1 | 0,19 | 0,19 |
| | Stamm 2 | 0,05 | 0,19 |
| Acinetobacter anitratus | | 25 | 100 |
| Proteus mirabilis | Stamm 1 | 0,025 | 0,10 |
| | Stamm 2 | 0,39 | 0,78 |
| Proteus inconstans | | ≤0,006 | 0,025 |
| Proteus vulgaris | Stamm 1 | 0,10 | 0,39 |
| | Stamm 2 | 0,10 | 0,19 |
| | Stamm 3 | 0,05 | 0,19 |
| Proteus rettgeri | | ≤0,006 | 0,05 |
| Serratia marcescens | Stamm 1 | 0,39 | 1,6 |
| | Stamm 2 | 0,19 | 0,78 |

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten. Die Verbindungen der Formel I, worin R Wasserstoff darstellt, und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonische Kochsalzlösung, zubereitet. Die leicht hydrolysierbaren Ester bzw. Aether der Formel I und ihre Salze bzw. Hydrate kommen auch für die enterale Verabreichung in Betracht.

## Beispiel 1

Herstellung des Dinatriumsalzes des (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-en-2-carbonsäure-5-(S)-oxids

5 g Dinatriumsalz der (6R,7R)-7-[2-(2-Amino-4-thiazolyl)-2-(Z-methoxyimino)acetamido]-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure werden in 100 ml Wasser gelöst. Dieser Lösung wird während 1/2 Stunde eine Lösung von 1,85 g 3-Chlorperbenzoesäure (85%ig) in 50 ml Aethanol zugetropft, wobei die Temperatur bei 25-28$^{o}$C gehalten wird. Das Gemisch wird danach 1 Stunde bei 25$^{o}$C gerührt und anschliessend im Vakuum eingeengt. Die resultierende Suspension wird zwischen Wasser und Aethylacetat verteilt. Die wässrige Lösung wird einmal mit Aethylacetat gewaschen und im Vakuum bei 50$^{o}$C eingedampft. Der Rückstand wird in 50 ml Wasser gelöst und unter Rühren langsam mit Aceton bis zur beginnenden Trübung versetzt. Durch Kratzen mit einem Glasstab wird die Kristallisation der Titelsubstanz induziert. Nach 30-minütigem Rühren wird das Kristallisat abgenutscht und nacheinander mit 100 ml Aceton/Wasser 8:2, 100 ml Aceton und 100 ml tiefsiedendem Petroläther gewaschen. Man erhält reine Titelsubstanz als praktisch farblose Kristalle mit $[\alpha]_D^{25}$ = -117$^{o}$ (c = 1 in Wasser).
$^{1}$H-NMR-Spektrum in $D_2O$ (δ-Werte in ppm, s = Singlett, d = Dublett, q = Quartett; J = Kopplungskontante in Hz, Protonenzahl in Klammern): 3,65 ($N-CH_3$)(s)(3), 3,87 ($2-CH_2$)(AB-q/Zentrierungswert; $J_{gem}$ = 18 Hz)(2), 4,03 [= $N^{\swarrow OCH_3}$,(Z)](s)(3), 4,29 ($3-CH_2-S$)(AB-q/Zentrierungswert; $J_{gem}$ = 13 Hz)(2), 5,00 (H-6)(d,$J_{H-6,H-7}$ = 4,5 Hz)(1), 6,00 (H-7)(d,$J_{H-7,H-6}$ = 4,5 Hz)(1), 7,02 (Thiazolyl-H)(s)(1).

Das als Ausgangsprodukt verwendete Dinatriumsalz der (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure kann wie folgt hergestellt werden:

22,24 g 2-(2-Chloracetamido-thiazol-4-yl)-2-[(Z)-methoxyimino]-essigsäure werden in 240 ml Methylenchlorid suspendiert. Dieser Suspension werden 13,39 ml Triäthylamin zugegeben, wobei eine hellbraune Lösung entsteht. Diese Lösung wird auf 0-5°C gekühlt und mit 16,72 g Phosphorpentachlorid versetzt, 5 Minuten bei 0-5°C und 20 Minuten ohne Kühlung gerührt. Die gelbe Lösung wird am Vakuum bei 35°C eingedampft. Der Eindampfrückstand wird zweimal mit n-Heptan geschüttelt und letzteres abdekantiert. Der harzige Rückstand wird mit 240 ml Tetrahydrofuran behandelt, und das ungelöste Triäthylamin-hydrochlorid wird abfiltriert. Das gelbe Filtrat enthält das Säurechlorid.

22 g (7R)-7-Amino-3-desacetoxy-3-[(2,5-dihydro-6-hydroxy-2-methyl-5-oxo-as-triazin-3-yl)-thio]-cephalosporansäure werden in einem Gemisch von 300 ml Wasser und 150 ml Tetrahydrofuran suspendiert. Zur Suspension wird unter guter Stickstoff-Begasung mit Hilfe des Autotitrators 2N Natronlauge zugetropft, bis eine braunrote Lösung von pH 8 entsteht. Diese wird auf 0-5°C gekühlt und während 15 Minuten tropfenweise mit der oben hergestellten Lösung des Säurechlorids in Tetrahydrofuran versetzt. Danach wird 2 1/2 Stunden bei 25°C gerührt. Das pH des Acylierungsgemisches wird unter Zugabe von 2N Natronlauge konstant bei 8 gehalten. Die praktisch schwarze Lösung wird am Vakuum bei 40°C von Tetrahydrofuran befreit. Nun werden 100 ml 2N Schwefelsäure zugegeben. Die dabei ausgefallene Substanz wird abgenutscht, mit Wasser gewaschen und gut abgesaugt. Das feuchte, braune

Nutschgut wird in 1,5 l Aceton gelöst. Die dunkle Lösung wird von wenig dunklem ungelöstem Material über Hyflo abfiltriert, mit Kohle versetzt, 30 Minuten gerührt und wieder über Hyflo filtriert. Das orange-rote Filtrat wird über Natriumsulfat getrocknet, am Vakuum eingeengt und mit Aethylacetat abgedampft. Dabei fällt ein schwarzes Harz aus, das abfiltriert und verworfen wird. Das 2-phasige, noch Wasser enthaltende Filtrat wird dreimal mit Benzol am Vakuum bei 40°C azeotropiert. Die dabei ausgefallene Substanz wird abgenutscht und im Vakuum bei 40°C getrocknet. Letztere wird zweimal mit je 1 l Aceton verrührt, wobei ein braunes Harz zurückbleibt, das verworfen wird. Die vereinigten orangefarbigen Acetonextrakte werden am Vakuum bei 40°C auf ca. 150 ml eingeengt, wobei ein braunes Harz abfiltriert und verworfen wird. Das Filtrat wird mit 1 Liter Aethylacetat versetzt und am Vakuum bei 40°C eingeengt. Die dabei ausgefallene Substanz wird abgenutscht, mit Aethylacetat und danach mit Aether gewaschen [(6R,-7R)-7-/2-[2-(2-Chloracetamido)-4-thiazolyl]-2-[(Z)-methoxyimino]acetamido/-3-/[(2,5-dihydro-6-hydroxy-2-methyl-5-oxo-as-triazin-3-yl)thio]-methyl-8-oxo-5-thia-1-azabicyclo[4.2.0]-oct-2-en-2-carbonsäure, Fraktion 1: eine beige, amorphe Säure]. Diese Fraktion 1 kann direkt für die Herstellung der gewünschten Ausgangsverbindung eingesetzt werden.

Die Aethylacetatmutterlauge wird am Vakuum bei 40°C stark eingeengt, mit Aether verdünnt und die ausgefallene Substanz abgenutscht [(6R,7R)-7-/2-[2-(2-Chloracetamido)-4-thiazolyl]-2-[(Z)-methoxyimino]acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure, Fraktion II: eine hellbeige amorphe Säure, dünnschichtchromatographisch etwas reiner als Fraktion I].

Zur Herstellung des Dinatriumsalzes werden 3,5 g Säure (Fraktion II) in einem Gemisch von 20 ml Aceton und 11 ml Wasser gelöst. Die Lösung wird mit 7 ml einer

2N Lösung von 2-Aethylcapronsäure-Natriumsalz in Aethylacetat versetzt, wobei das Dinatriumsalz kristallisiert. Nun werden noch portionenweise 25 ml Aceton hinzugefügt und das Gemisch 2 Stunden im Tiefkühlschrank aufbewahrt. Danach wird das Kristallisat abgenutscht, nacheinander mit 25 ml eines eiskalten Aceton-Wasser-Gemisches (80:20), reinem Aceton und tiefsiedendem Petroläther gewaschen und über Nacht im Hochvakuum bei 40°C getrocknet. Man erhält das Dinatriumsalz der (6R,7R)-7-/2-[2-(2-Chloracetamido)-4-thiazolyl]-2-[(Z)-methoxyimino]-acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure als hellgelbe Kristalle. $[\alpha[_D^{20} = -142,7^o$ (c = 1 in Wasser). Das Kernresonanzspektrum und die Mikroanalyse entsprechen der angegebenen Struktur.

15,3 g (6R,7R)-7-/2-[2-(2-Chloracetamido)-4-thiazolyl]-2-[(Z)-methoxyimino]acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-en-2-carbonsäure (Fraktion I) werden zusammen mit 5 g Thioharnstoff in 150 ml Wasser suspendiert. Unter guter Stickstoff-Begasung und Rühren wird das pH mit gesättigter Natriumhydrogencarbonatlösung auf 6,8-7,0 gestellt, wobei eine orangefarbige Lösung entsteht. Mittels Autotitrator unter Zugabe von Natriumhydrogencarbonatlösung wird das pH der Reaktionslösung konstant bei 6,8 während 6 Stunden gehalten. Danach werden noch 2,5 g Thioharnstoff hinzugegeben, und die Lösung wird weitere 3 Stunden gerührt, wobei das pH unter Zugabe von gesättigter Natriumhydrogencarbonatlösung bei 6,8 gehalten wird. Danach wird die rote Lösung über Nacht im Kühlschrank aufbewahrt, wobei diese dunkler wird. Das pH dieser Lösung wird durch Zugabe von 100%iger Ameisensäure auf 2,0-2,5 eingestellt, wobei die Substanz ausfällt. Diese wird abgenutscht und mit 100 ml 10%iger wässriger Ameisensäure gewaschen. Die Mutterlauge wird verworfen. Das bräunliche Nutschgut wird in 200 ml Wasser

suspendiert und das pH mit Triäthylamin auf 7 gestellt, wobei eine braune Lösung entsteht. Diese Lösung wird mit 2 g Aktivkohle 30 Minuten gerührt, von der Kohle abfiltriert und das immer noch braune Filtrat mit 100%iger Ameisensäure unter gutem Rühren auf pH 3,5 gestellt. Die dabei ausgefallene Substanz wird abgenutscht, mit 50 ml 10%iger wässriger Ameisensäure gewaschen und verworfen. Das dunkelgelbe Filtrat wird mit 100%iger Ameisensäure auf pH 2-2,5 gestellt, wobei die Substanz ausfällt. Diese wird abgenutscht, mit Eiswasser gewaschen und getrocknet. Die erhaltene Cephalosporin-Säure wird, zwecks Ueberführung in das Dinatriumsalz, in einem Gemisch von 40 ml Aceton und 40 ml Wasser suspendiert und mit 20 ml einer 2N Lösung von 2-Aethylcapronsäure-Natriumsalz in Essigester versetzt. Zur dabei entstandenen orangefarbigen Lösung werden 50 ml Aceton gegeben, wobei ein braunes Harz ausfällt, das mittels Filtration abgetrennt wird. Das gelbe Filtrat wird 30 Minuten gerührt, wobei das Dinatriumsalz kristallisiert. Das Gemisch wird noch portionenweise mit 50 ml Aceton versetzt und über Nacht im Kühlschrank aufbewahrt. Das Kristallisat wird abgenutscht, nacheinander mit einem Aceton-Wasser-Gemisch (85:15), reinem Aceton und tiefsiedendem Petroläther gewaschen und über Nacht im Vakuum bei 40°C getrocknet. Man erhält das Dinatriumsalz der (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure als beige-farbene Kristalle. $[\alpha]_D^{20} = -144^\circ$ (c = 0,5 in Wasser). Das Kernresonanzspektrum und die Mikroanalyse entsprechen der angegebenen Struktur.

## Beispiel 2

Herstellung von Methylen (6R,7R)-7-/2-(2-Amino-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]-oct-2-en-2-carboxylat-pivalat-5-(S)-oxid

0,875 g Methylen (6R,7R)-7-/2-(2-Chloracetamido-4-thiazolyl)-2[(Z)-methoxyimino]acetamido/-3-/[(2,5-dihydro-2-methyl-5-oxo-6[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid werden in 15 ml N,N-Dimethylacetamid zusammen mit 0,500 g Thioharnstoff gelöst. Die Lösung wird 2 Stunden bei 25°C gerührt und anschliessend auf 200 ml Aethylacetat gegossen. Das Gemisch wird einmal mit 5%iger wässriger Natriumhydrogencarbonat-Lösung und zweimal mit Wasser gewaschen. Die organische Phase wird über Natriumsulfat getrocknet und im Vakuum bei 40°C eingedampft. Der bräunliche Eindampfrückstand wird zur Reinigung an einer Kieselgelsäule mit den Elutionssystemen Benzol/Aethanol 9:1 und Benzol/Aethanol 8:2 chromatographiert. Die die Titelsubstanz enthaltenden Fraktionen werden gesammelt und am Vakuum bei 40°C stark eingeengt. Das Konzentrat wird mit Aether versetzt, wobei hellbeige Titelsubstanz amorph ausfällt.
$^1$H-NMR-Spektrum in DMSO-$d_6$ ($\delta$-Werte in ppm, s = Singlett, d = Dublett, q = Quartett, m = Multiplett, b = breit, J = Kopplungskonstante in Hz; Protonenzahl in Klammern): 1,18 [2x C $(CH_3)_3$](s)(18), 3,63 (N-$CH_3$)(s)(3), 3,86 [=N$\swarrow$$OCH_3$, (Z)](s)(3), 4,45 (3-$CH_2$-S)(AB-q/Zentrierungswert; $J_{gem}$ = 14 Hz)(2), 4,96 (H-6)(d, $J_{H-6,H-7}$ = 5 Hz)(1), 5,85 (2x O-$CH_2$-O)(m)(4), 6,03 (H-7)(q, $J_{H-7,NH}$ = 8 Hz, $J_{H-7,H-6}$ = 5 Hz)(1), 6,82 (Thiazolyl-H)(s)(1), 7,17 (-$NH_2$)(b)(2), 8,77 (7-NH)(d,$J_{NH,H-7}$ = 8 Hz)(1).

Das als Ausgangsprodukt verwendete Methylen (6R,-7R)-7-/2-(2-Chloracetamido-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid kann wie folgt hergestellt werden:

13,5 g Dinatriumsalz der (6R,7R)-7-/2-(2-Chloracetamido-4-thiazolyl)-2-[(Z)-methoxyimino]acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure werden in 400 ml Dimethylformamid suspendiert. Die Suspension wird auf 0-5°C gekühlt, 15 Minuten gerührt und mit 8,8 g Pivaloyloxymethyljodid versetzt. Das Gemisch wird 30 Minuten bei 0-5°C gerührt, anschliessend auf 2 l Aethylacetat gegossen und nacheinander mit 400 ml Wasser, zweimal mit je 600 ml 1M wässriger Natriumhydrogencarbonat-Lösung und zweimal mit je 1 l Wasser gewaschen. Die organische Phase wird danach über Natriumsulfat getrocknet und am Vakuum bei 40°C auf ein Volumen von ca. 50 ml konzentriert. Dieses Konzentrat wird unter Rühren mit Aether versetzt, wobei das Rohprodukt ausfällt. Nach dem Umfällen aus Aethylacetat/Aether und Trocknen über Nacht am Vakuum bei 25°C erhält man vorgereinigtes Produkt, das zur weiteren Reinigung an einer Kieselgelsäule mit Aethylacetat als Elutionsmittel chromatographiert wird. Die das Produkt enthaltenden Fraktionen werden gesammelt und eingedampft. Der Rückstand wird aus Aethylacetat/-Aether kristallisiert und liefert reines, beiges Methylen (6R,7R)-7-/2-(2-Chloracetamido-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat mit $[\alpha]^{25}_{D} = -202,7^{o}$ (c = 0,7837 in Aceton).
$^{1}$H-NMR-Spektrum in DMSO-$d_6$ ($\delta$-Werte in ppm, s = Singlett, d = Dublett, q = Quartett, m = Multiplett, J = Kopplungskonstante in Hz, Protonenzahl in Klammern): 1,15 [2x $C(CH_3)_3$](s)(18), 3,61 ($N-CH_3$)(s)(3), ca. 3,66 ($2-CH_2$)(AB-q/Zentrierungswert; $J_{gem}$ = ca. 12 Hz)(2), 3,85 [= $N\diagup^{OCH_3}$, (Z)](s)(3), 4,25 ($3-CH_2-S$)(AB-q/Zentrierungswert, $J_{gem}$ = 14 Hz), 4,34 ($Cl-CH_2-CO$)(s)(2), 5,15 (H-6)(d, $J_{H-6,H-7}$ = 4,5 Hz)(1), 5,81 (2x $O-CH_2-O$)

(m)(4), 5,95 (H-7)(q, $J_{H-7,NH}$ = 8,5 Hz), $J_{H-7,H-6}$ = 4,5 Hz)(1), 7,39 (Thiazolyl-H)(s)(1), 9,59 (7-NH)(d, $J_{NH,H-7}$ = 8,5 Hz)(1), 12,81 (N<u>H</u>-COCH$_2$Cl)(s)(1).

5,5 g Methylen (6R,7R)-7-/2-(2-Chloracetamido-4-thiazolyl)-2-[(Z)-methoxyimino]acetamido/-3-/[(2,5-dihydro-2-methyl-5-oxo-6-[(pivaloyloxy)methoxy]-as-triazin-3-yl)-thio]methyl/-8-oxo-5-thia-1-azabicyclo[4.2.0]oct-2-en-2-carboxylat-pivalat werden in 25 ml Chloroform gelöst. Der auf 0°C abgekühlten Lösung wird während 20 Minuten eine Lösung von 1,34 g 3-Chlorperbenzoesäure (85%ig) in 20 ml Chloroform zugetropft, wobei die Temperatur der Reaktionslösung zwischen 3°C und 5°C gehalten wird. Danach wird die Reaktionslösung 15 Minuten bei 5°C gerührt und anschliessend mit 500 ml Aethylacetat verdünnt. Nach dem Waschen mit wässriger Natriumhydrogencarbonat-Lösung und mit Wasser wird die organische Phase am Vakuum bei 40°C eingedampft. Als Rückstand verbleibt ein braunes Pulver, welches zur Reinigung an einer Kieselgelsäule mit Aethylacetat/Methanol 9:1 als Elutionsmittel chromatographiert wird. Die das Produkt enthaltenden Fraktionen werden vereinigt und am Vakuum bei 40°C eingedampft. Der Rückstand wird in wenig Aethylacetat gelöst und durch Versetzen mit Aether gefällt. Der amorphe Niederschlag wird abgenutscht und nacheinander mit Aether und tiefsiedendem Petroläther gewaschen. Man erhält reines Methylen (6R,7R)-7-/2-(2-Chloracetamido-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid als praktisch farbloses Pulver. $^1$H-NMR-Spektrum in DMSO-d$_6$ ($\delta$-Werte in ppm, s = Singlett, d = Dublett, q = Quartett, J = Kopplungskonstante in Hz; Protonenzahl in Klammern): 1,19 [2x C(CH$_3$)$_3$] (s)(18), 3,66 (N-CH$_3$)(s)(3), ca.3,70 (2-CH$_2$)(AB-q/Zentrierungswert; $J_{gem}$ = ca. 18 Hz)(2), 3,93 [=N$^{\nearrow OCH_3}$, (Z)](s)(3), 4,37 (Cl-CH$_2$-CO)(s)(2), ca. 4,50 (3-CH$_2$-S)

(AB-q/Zentrierungswert; $J_{gem}$ = 15 Hz)(2), 5,00 (H-6) (d, $J_{H-6,H-7}$ = 4,5 Hz)(1), 5,87 (2x O-$CH_2$-O)(s, breit) (4), 6,07 (H-7)(q, $J_{H-7,NH}$ = 7,5 Hz, $J_{H-7,H-6}$ = 4,5 Hz) (1), 7,50 (Thiazolyl-H)(s)(1), 9,03 (7-NH)(d, $J_{NH,H-7}$ = 7,5 Hz)(1), 12,98 (N<u>H</u>-$COCH_2Cl$)(s)(1).

## Beispiel 3

Herstellung des Dinatriumsalzes des (6R,7R)-7-/2-(2-Amino-4-thiazolyl)-2-[(Z)-methoxyimino]acetamido/-3-/[(2,5-dihydro-6-hydroxy-2-methyl-5oxo-as-triazin-3-yl)-thio]methyl/-8-oxo-5-thia-1-azabicyclo[4.2.0]oct-2-en-2-carbonsäure-5,5-dioxids

1,8 g Dinatriumsalz des (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxids werden in 35 ml Wasser gelöst. Dieser Lösung wird während 20 Minuten eine Lösung von 0,627 g 3-Chlorperbenzoesäure (85%ig) in 17 ml Aethanol zugetropft, wobei die Reaktionstemperatur bei 25-28°C gehalten wird. (Der Verbrauch an Oxidationsmittel wird mittels Kaliumjodid-Stärke-Papier verfolgt). Danach wird das Reaktionsgemisch 90 Minuten bei 25°C gerührt und anschliessend am Vakuum bei 45°C eingeengt. Das Konzentrat wird zwischen Wasser und Aethylacetat verteilt. Die wässrige Phase wird einmal mit Aethylacetat gewaschen und am Vakuum bei 45°C eingedampft. Der verbleibende Rückstand liefert nach dem Pulverisieren und Trocknen beige-gelbe Titelverbindung. $^1$H-NMR-Spektrum in $D_2O$ ($\delta$-Werte in ppm, s = Singlett, d = Dublett, b = breit, Protonenzahl in Klammern): 3,71 (N-$CH_3$)(s,b)(3), 4,02 [= $N^{\nearrow OCH_3}$,(Z)](s)(3), 4,99 (H-6) (d,b)(1), 6,01 (H-7)(d,b)(1), 7,03 (Thiazolyl-H)(s)(1).

## Beispiel 4

Herstellung von Trockenampullen für die intramuskuläre Verabreichung:

Es wird in üblicher Weise ein Lyophilisat von 1 g des Dinatriumsalzes des (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxids hergestellt und in eine Ampulle abgefüllt. Vor der Verabreichung wird das Lyophilisat mit 2,5 ml einer 2%igen wässrigen Lidocainhydrochlorid-Lösung versetzt.

## Beispiel 5

Es wird in üblicher Weise eine Gelatine-Steckkapsel folgender Zusammensetzung hergestellt:

| | |
|---|---|
| Methylen (6R,7R)-7-/2-(2-Amino-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

Patentansprüche

1. Cephalosporinderivate der allgemeinen Formel

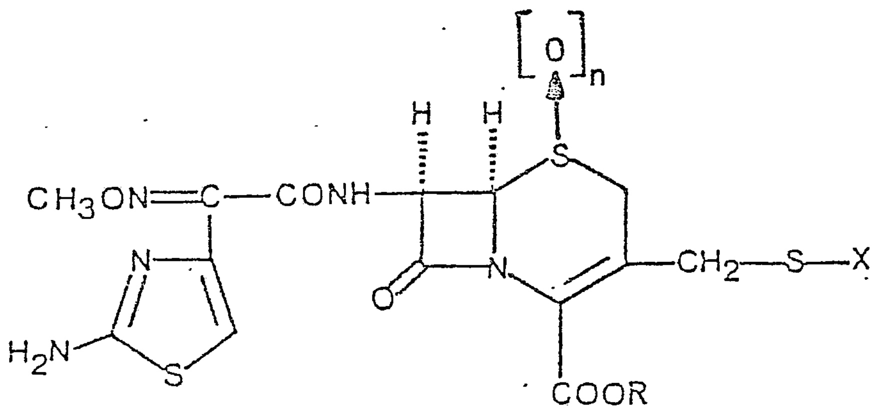

I

in der X eine der Gruppen

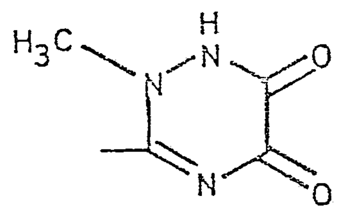

(a)

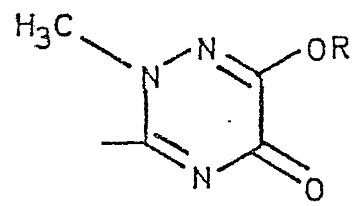

(b)

bedeutet, R Wasserstoff oder eine leicht hydrolysierbare Ester- bzw. Aethergruppe und n die Zahl 1 oder 2 darstellt,
sowie Salze dieser Verbindungen und Hydrate dieser Verbindungen bzw. Salze.

2. Derivate nach Anspruch 1, dadurch gekennzeichnet, dass R Wasserstoff darstellt.

3. Derivate nach Anspruch 1 oder 2, dadurch gekennzeichnet, dass n die Zahl 1 darstellt.

4. Derivate nach Anspruch 3, dadurch gekennzeichnet, dass das Sauerstoffatom der Sulfoxidgruppe in ß-Stellung (S-Konfiguration) vorliegt.

5. (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid sowie Salze dieser Verbindung und Hydrate dieser Verbindung bzw. Salze.

6. (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5,5-dioxid sowie Salze dieser Verbindung und Hydrate dieser Verbindung bzw. Salze.

7. Pivaloyloxymethylester bzw. -äther der Formel I gemäss Anspruch 1 sowie Salze dieser Verbindungen und Hydrate dieser Verbindungen bzw. Salze.

8. Methylen (6R,7R)-7-/2-(2-Amino-4-thiazolyl)-2-[(Z)-methoxyimino]acetamido/-3-/[(2,5-dihydro-2-methyl-5-oxo-6-[(pivaloyloxy)methoxy]-as-triazin-3-yl)thio]methyl/-8-oxo-5-thia-1-azabicyclo[4.2.0]oct-2-en-2-carboxylat-pivalat-5-(S)-oxid sowie Salze dieser Verbindung und Hydrate dieser Verbindung bzw. Salze.

9. Cephalosporinderivate der allgemeinen Formel

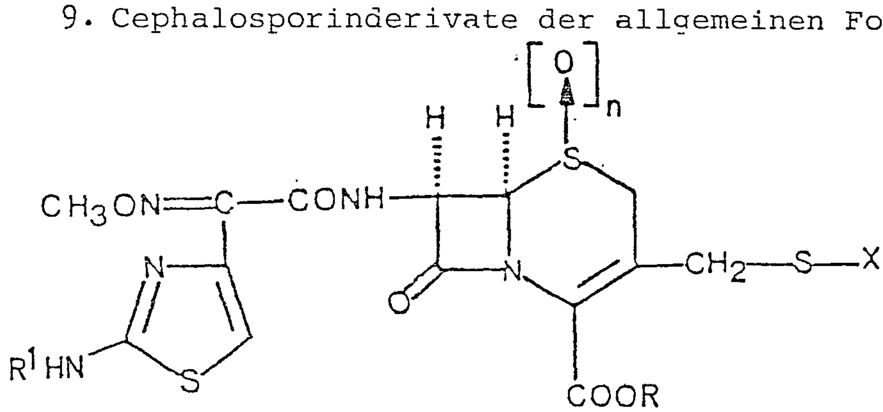

III

in der X, R und n die in Anspruch 1 gegebene Bedeutung haben und $R^1$ eine abspaltbare Schutzgruppe darstellt,

und Salze dieser Verbindungen.

10. Halogenide der allgemeinen Formel

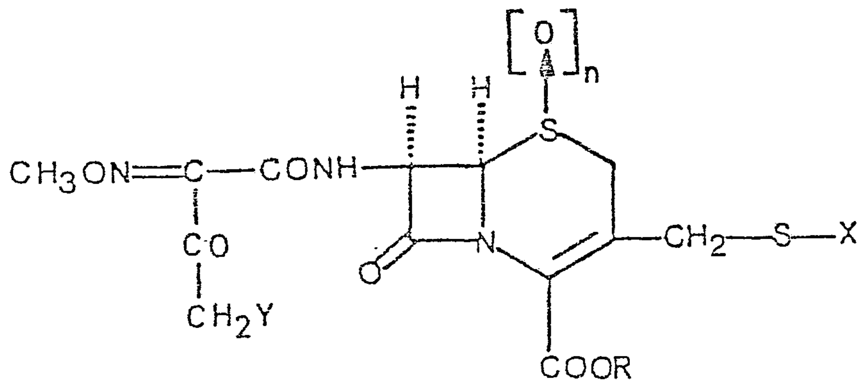

IV

in der X, R und n die in Anspruch 1 gegebene Bedeutung haben und Y ein Halogenatom darstellt,

und Salze dieser Verbindungen.

11. Cephalosporinderivate der allgemeinen Formel

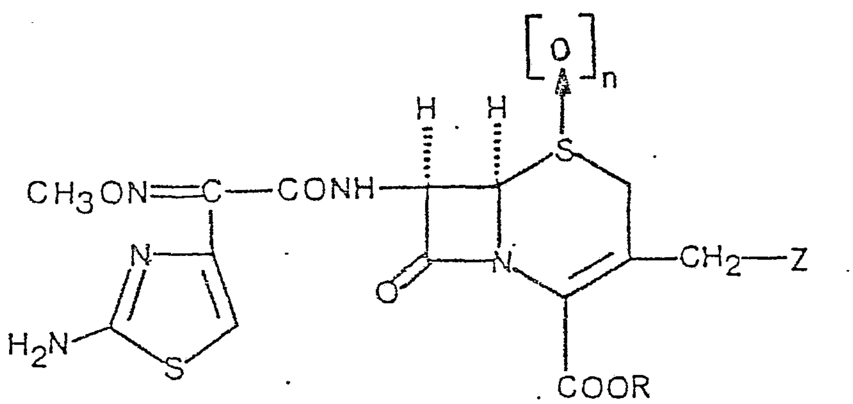

V

in der R und n die in Anspruch 1 gegebene Bedeutung haben und Z eine austretende Gruppe darstellt,

und Salze dieser Verbindungen.

12. Verbindungen gemäss einem der Ansprüche 1-8 als pharmazeutische Wirkstoffe.

13. (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid sowie Salze dieser Verbindung und Hydrate dieser Verbindung bzw. Salze als pharmazeutische Wirkstoffe.

14. Verbindungen gemäss einem der Ansprüche 1-8 als pharmazeutische Wirkstoffe zur Behandlung und Prophylaxe von Infektionskrankheiten.

15. (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid sowie Salze dieser Verbindung und Hydrate dieser Verbindung bzw. Salze als pharmazeutische Wirkstoffe zur Behandlung und Prophylaxe von Infektionskrankheiten.

16. Pharmazeutische Präparate gekennzeichnet durch einen Gehalt an einer Verbindung gemäss einem der Ansprüche 1-8.

17. Präparat nach Anspruch 16, dadurch gekennzeichnet, dass es (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid, ein Salz dieser Verbindung oder ein Hydrat dieser Verbindung bzw. dieses Salzes enthält.

18. Pharmazeutische Präparate zur Behandlung und Prophylaxe von Infektionskrankheiten, gekennzeichnet durch einen Gehalt an einer Verbindung gemäss einem der Ansprüche 1-8.

19. Präparat nach Anspruch 18, dadurch gekennzeichnet, dass es (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid, ein Salz dieser Verbindung oder ein Hydrat dieser Verbindung bzw. dieses Salzes enthält.

20. Verfahren zur Herstellung der Cephalosporinderivate gemäss einem der Ansprüche 1-8, dadurch gekennzeichnet, dass man

a) eine Verbindung der allgemeinen Formel

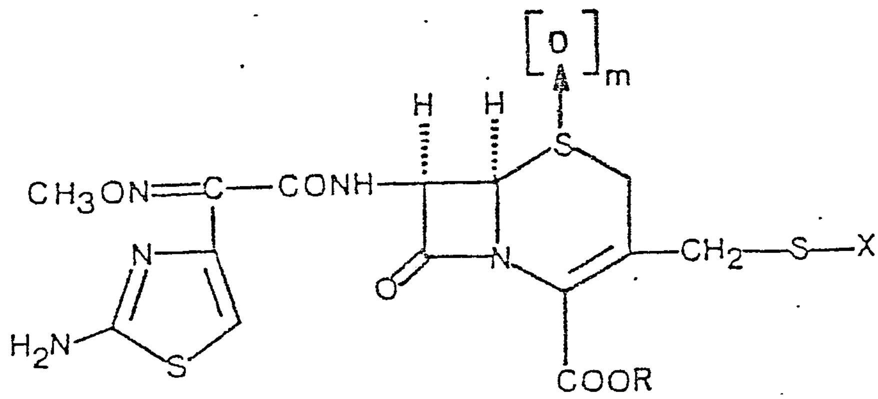

II

in der X und R die in Anspruch 1 gegebene Bedeutung haben und m die Zahl 0 oder 1 darstellt,

oder ein Salz dieser Verbindung oxidiert, oder dass man

b) in einer Verbindung der allgemeinen Formel

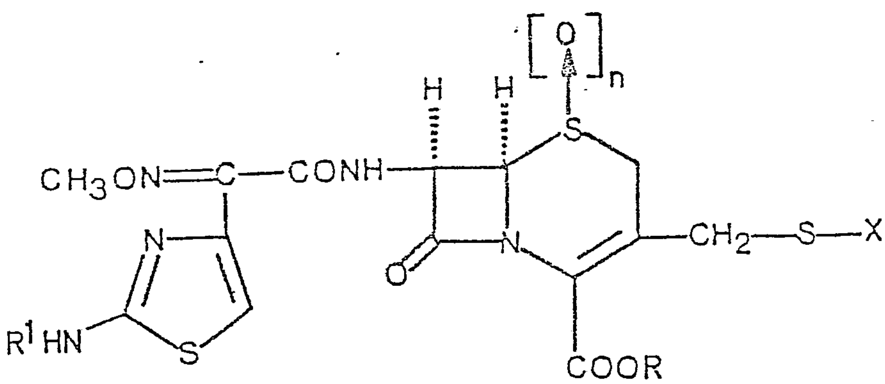

III

in der X, R und n die in Anspruch 1 gegebene Bedeutung haben und $R^1$ eine abspaltbare Schutzgruppe darstellt,

oder in einem Salz dieser Verbindung die Schutzgruppe $R^1$ abspaltet, oder dass man

c) ein Halogenid der allgemeinen Formel

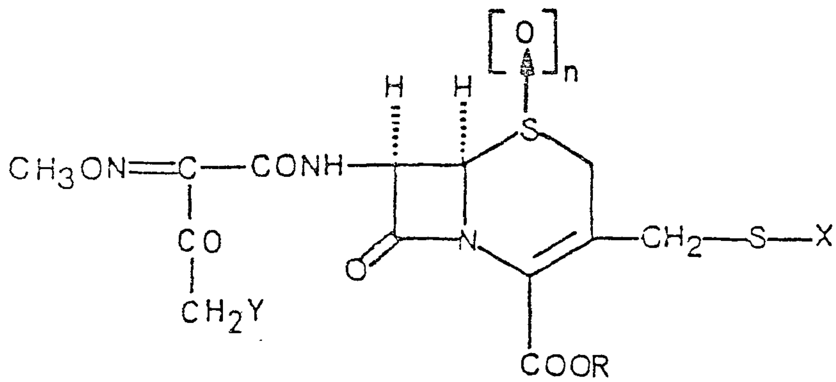

IV

in der X, R und n die in Anspruch 1 gegebene Bedeutung haben und Y ein Halogenatom darstellt, oder ein Salz dieser Verbindung mit Thioharnstoff umsetzt, oder dass man

d) eine Verbindung der allgemeinen Formel

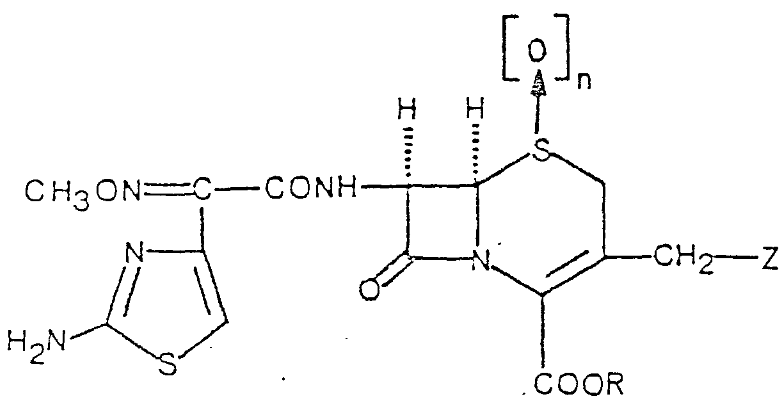

V

in der R und n die in Anspruch 1 gegebene Bedeutung haben und Z eine austretende Gruppe darstellt, oder ein Salz dieser Verbindung mit einem Thiol der allgemeinen Formel

HS-X VI

in der X die in Anspruch 1 gegebene Bedeutung hat, umsetzt, oder dass man

e) zur Herstellung von leicht hydrolysierbaren Estern bzw. Aethern der Formel I, d.h. worin R eine leicht hydrolysierbare Ester- bzw. Aethergruppe darstellt, eine Carbonsäure bzw. ein Enol der Formel I einer entsprechenden Veresterung bzw. Verätherung unterwirft, oder dass man

f). zur Herstellung von Salzen und Hydraten einer Verbindung der Formel I bzw. von Hydraten dieser Salze eine Verbindung der Formel I in ein Salz oder Hydrat bzw. ein Hydrat dieses Salzes überführt.

21. Verwendung von Verbindungen gemäss einem der Ansprüche 1-8 bei der Behandlung bzw. Prophylaxe von Krankheiten.

22. Verwendung von (6R,7R)-7-/2-(2-Amino-4-thiazolyl)-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-én-2-carbonsäure-5-(S)-oxid sowie von Salzen dieser Verbindung und von Hydraten dieser Verbindung bzw. Salze bei der Behandlung bzw. Prophylaxe von Krankheiten.

23. Verwendung von Verbindungen gemäss einem der Ansprüche 1-8 bei der Behandlung bzw. Prophylaxe von Infektionskrankheiten.

24. Verwendung von (6R,7R)-7-/2-(2-Amino-4-thiazolyl-2-[(Z)-methoxyimino]acetamido/-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure-5-(S)-oxid sowie von Salzen dieser Verbindung und von Hydraten dieser Verbindung bzw. Salze bei der Behandlung bzw. Prophylaxe von Infektionskrankheiten.

****